# EUROPÄISCHE PATENTSCHRIFT

(11) **EP 1 056 467 B1**
(45) Veröffentlichungstag und Bekanntmachung des Hinweises auf die Patenterteilung: **01.10.2003**
(21) Anmeldenummer: 99910259.3
(22) Anmeldetag: 23.02.1999
(51) Int. Cl.: A61K 38/00, A61P 27/16

(54) **VERFAHREN ZUR BEHANDLUNG VON ERKRANKUNGEN ODER STÖRUNGEN DES INNENOHRS**
METHOD FOR THE TREATMENT OF DISEASES OR DISORDERS OF THE INNER EAR
PROCEDE POUR LE TRAITEMENT DE MALADIES OU DE TROUBLES DE L'OREILLE INTERNE

(30) Priorität: 23.02.1998 DE 19807426
(43) Veröffentlichungstag der Anmeldung: 06.12.2000
(73) Patentinhaber: Sound Pharmaceuticals Incorporated, Seattle, WA 98103 (US)
(72) Erfinder: LÖWENHEIM, Hubert, D-72076 Tübingen (DE)
(74) Vertreter: Patentanwälte Ruff, Wilhelm, Beier, Dauster & Partner
(86) Internationale Anmeldenummer: EP9901153
(87) Internationale Veröffentlichungsnummer: WO99042088

(56) Entgegenhaltungen:
- WO-A-97/17983
- WO-A-97/22255
- WO-A-98/00014
- WO-A-98/13048
- WO-A-99/06064
- CHEN, P. ET AL: "p27/Kip1, a cyclin -dependent kinase inhibitor, is required for the normal development of the mouse auditory sense organ." SOCIETY FOR NEUROSCIENCE ABSTRACTS, (1998) VOL. 24, NO. 1-2, PP. 809. MEETING INFO.: 28TH ANNUAL MEETING OF THE SOCIETY FOR NEUROSCIENCE, PART 1 LOS ANGELES, CALIFORNIA, USA NOVEMBER 7-12, 1998 SOCIETY FOR NEUROSCIENCE. , XP002123936
- SCHWEITZER V G: "Cisplatin-induced ototoxicity: the effect of pigmentation and inhibitory agents." LARYNGOSCOPE, (1993 APR) 103 (4 PT 2) 1-52. REF: 151 , XP002123937
- UMEMOTO M ET AL: "HAIR CELL REGENERATION IN THE CHICK INNER EAR FOLLOWING ACOUSIC TRAUMA: ULTRASTRUCTURAL AND IMMUNOHISTOCHEMICAL STUDIES" CELL AND TISSUE RESEARCH,DE,BERLIN, Bd. 3, Seite 435-443 XP002060017
- BUJIA J. ET AL: "Immunohistochemical detection of proliferating cell nuclear antigen in middle ear cholesteatoma." EUROPEAN ARCHIVES OF OTO-RHINO-LARYNGOLOGY, (1996) 253/1-2 (21-24). , XP002123938
- YAMAGUCHI T ET AL: "The effect of chalone on the cell cycle in the epidermis during wound healing." EXPERIMENTAL CELL RESEARCH, (1974 DEC) 89 (2) 247-54. , XP002123939
- COATS S. ET AL: 'Requirement of p27Kip1 for Restriction Point Control of the Fibroblast' SCIENCE Bd. 271, 1996, Seiten 877 - 880
- ST. CROIX B. ET AL: 'impact of the cyclin-dependent kinase inhibitor p27kip1 on resistance of tumor cells to anticancer agents' NATURE MEDICINE Bd. 2, 1996, Seiten 1204 - 1210
- FIELDS S. ET AL: 'the two-hybrid system: an asssay for protein-protein interactions' TRENDS GENET Bd. 10, Nr. 8, 1994, Seiten 286 - 292
- RUSSO A. ET AL: 'Crystal structure of the p27Kip1 cyclin-dependent-kinase inhibitor bound to the cyclin A-Cdk2 complex' NATURE Bd. 382, 1996, Seiten 325 - 331
- CHARDIN S. ET AL: 'Regeneration and Mammalian Auditory hair cells' SCIENCE Bd. 267, 1995, Seiten 707 - 709

## Beschreibung

Die Erfindung betrifft die Verwendung eines Wirkstoffs zur Herstellung einer pharmazeutischen Zusammensetzung

Das Innenohr des Menschen und anderer Säugetiere kann entweder von vorneherein durch einen genetischen Defekt oder später durch äußere Einflüsse irreversibel geschädigt sein. Bei den genannten äußeren Einflüssen kann es sich beispielsweise um Schalltraumata oder um toxische oder hypoxische Einflüsse handeln. Diese Schädigungen können in Funktionsstörungen oder Funktionsausfällen der im Innenohr lokalisierten Sinne, insbesondere des Hörens, resultieren. Bei diesen Funktionsstörungen ist insbesondere eine Reduktion oder ein Schwinden des Hörvermögens zu nennen. Schätzungsweise dürften in Deutschland ca. 12 Millionen Menschen an einer sogenannten Innenohrschwerhörigkeit leiden, die auf die genannten Pathomechanismen zurückzuführen ist. Als Ursache für den teilweisen oder vollständigen Verlust des Hörvermögens kommt hier neben der Degeneration von sensorischen Neuronen und Schädigungen der sogenannten Stria vascularis des Innenohrs, insbesondere eine Schädigung oder Zerstörung der Sinneszellen des Innenohrs und damit des Hörorgans in Frage.

Bei einem Verfahren zur Behandlung von Erkrankungen oder Störungen des Innenohrs, die mit einer Schädigung oder Zerstörung von Sinneszellen im Zusammenhang stehen, ist zu berücksichtigen, daß in den hochdifferenzierten Sinnesepithelien im Innenohr des Menschen und anderer Säuger irreversibel geschädigte und damit verlorene Zellen nicht mehr regeneriert werden können (Corwin J.T., Cotanche D.A. (1988) Science 240, 1772-4) Ein teilweiser oder vollständiger Hörverlust aufgrund Schädigung oder Zerstörung von Sinneszellen des Innenohrs bleibt damit in der Regel irreversibel. Hier unterscheiden sich die Sinnesepithelien des Innenohrs grundlegend von anderen Geweben, in denen abgestorbene Zellen durch die Teilung von Ersatzzellen und deren anschließende Ausdifferenzierung schnell ersetzt werden können.

Interessanterweise ist bei anderen Wirbeltierklassen wie beispielsweise den Vögeln im Gegensatz zu der Situation beim Menschen eine Regeneration abgestorbener Sinneszellen im Innenohr möglich. Hier werden die nach einer Schädigung abgestorbenen Sinneszellen durch sogenannte Stützzellen, die im Epithel unterhalb der Sinneszellen angeordnet sind, ersetzt. Dies erfolgt durch Teilung der Stützzellen und anschließende Ausdifferenzierung, wobei aus einer Stützzelle eine neue stützzelle und eine Sinneszelle hervorgeht(z.B. Ryals B.M., Rubel E.W. (1988) Science 240, 1774-6).

Die Entdeckung der Regeneration von Sinneszellen in der Cochlea des Vogels hat in den letzten Jahren zu dem Versuch geführt, Untersuchungsergebnisse beim Vogel auf Säugetiere und damit letztendlich auf den Menschen zu übertragen. Dies erschien unter anderem deshalb erfolgversprechend, da die Cochlea des Vogels und die Cochlea von Säugern zellbiologische Gemeinsamkeiten aufweisen. Sowohl das Sinnesepithel der Vogelcochlea als auch das Sinnesepithel der Säugercochlea sind nämlich postmitotisch, das heißt die in den Sinnesepithelien vorhandenen Sinneszellen werden nur während eines ganz bestimmten Zeitabschnitts in der embryonalen Entwicklung ausgebildet. Danach finden normalerweise keine weiteren Zellteilungen statt. Diese grundsätzliche Gemeinsamkeit macht aber die Erscheinung, daß sich im vestibulären Sinnesepithel des Vogels während seines gesamten Lebens Zellteilungen nachweisen lassen, beim Menschen jedoch nicht, schwer verständlich.

Da beim Vogel erkannt wurde, daß sogenannte Wachstumsfaktoren in der Vogelcochlea eine erhöhte Proliferationsrate hervorrufen können, wurden solche Wachstumsfaktoren auch in der Cochlea des Säugers eingesetzt. Es ließ sich jedoch keine reproduzierbare Wirkung nachweisen. Dies legt den Schluß nahe, daß trotz einer grundsätzlichen zellbiologischen Gemeinsamkeit, andere signifikante Unterschiede zwischen Vogelcochlea und Säugercochlea vorhanden sein müssen. Diese könnten darin liegen, daß die Stützzellen der Vogelcochlea zwar wie beim Säuger postmitotisch sind, den Zellzyklus jedoch nur vorübergehend verlassen haben. Sie können dann beim Auftreten eines entsprechenden Signals wieder in den Zellzyklus eintreten. Derartige Stützzellen können als quiescent, das heißt in Wartestellung stehend bezeichnet werden. Im Gegensatz dazu können die Stützzellen des Säugers eine sehr hohe und spezielle Differenzierung durchlaufen und damit den Zellzyklus irreversibel verlassen haben. Dementsprechend wären sie als terminal differenziert zu bezeichnen und beispielsweise den Neuronen vergleichbar. Dies kann für die Stützzellen des Säugers gelten, die als sogenannte Pillarzellen oder Deiterszellen bezeichnet werden. Solche Erklärungsmodelle für zellbiologische Unterschiede zwischen Vogelcochlea und Säugercochlea werden derzeit zum Anlaß genommen, die Regeneration der Sinneszellen beim Vogel noch detaillierter zu untersuchen, um später weitere dort erhaltene Ergebnisse auf den Säuger übertragen zu können.

Von Coats et al. (Science (1996), 272, 877 - 880) und Brad St. Croix et al. (Nature Medicine (1996), 2, 1204 - 1210) wurde die Verwendung von p27^{Kip1} Antisense-Oligonukleotiden bei der Untersuchung von Fibroblasten bzw. von Tumorzellen beschrieben. Coats et al. konnten beispielsweise bei Fibroblasten zeigen, dass durch den Einsatz von p27^{Kip1} Antisense-Oligonukleotiden der Übergang vom Zustand der Proliferation in den Zustand der Quieszenz, welcher durch Entzug von Wachstumsfaktoren induziert wird, verhindert werden kann.

Die Erfindung stellt sich demgegenüber die Aufgabe, einen neuen Ansatz zur Behandlung von Erkrankungen oder Störungen des Innenohrs, die mit einer Schädigung oder Zerstörung der Sinneszellen des Innenohrs im Zusammenhang stehen, zu finden. Hierbei soll weniger versucht werden, an anderen Wirbeltieren als den Säugern erhaltene Ergebnisse auf Säuger und insbesondere den Menschen zu übertragen, als vielmehr einen Wirkmechanismus und entsprechende Wirkstoffe zur Verfügung zu stellen, die direkt in die Zellvorgänge beim Säuger eingreifen und letztlich in einer Regeneration der Sinneszellen des Innenohrs resultieren.

Diese Aufgabe wird gelöst durch die Verwendung eines Wirkstoffs mit den Merkmalen des Anspruchs 1.

Bevorzugte Ausführungen sind in den abhängigen Ansprüchen 2 bis 5 dargestellt. Der Inhalt sämtlicher Ansprüche wird hiermit durch Bezugnahme zum Inhalt dieser Beschreibung gemacht.

Die Verwendung der eingangs genannten Art ermöglicht, daß mindestens ein im Innenohr vorhandener sogenannter Zellzyklus-Inhibitor in seiner inhibierenden Wirkung durch (mindestens) einen Wirkstoff mindestens teilweise gehemmt oder ausgeschaltet wird. Dies resultiert in einer Regeneration der Sinneszellen des Innenohrs.

Der Wirkstoff, der in der Lage ist, einen im Innenohr vorhandenen Zellzyklus-Inhibitor in seiner Wirkung zu hemmen oder auszuschalten, kann zur Herstellung einer pharmazeutischen Zusammensetzung oder eines Medikaments zur Behandlung von Erkrankungen oder Störungen des Innenohrs eingesetzt werden, wobei diese Erkrankungen/Störungen mit einer Schädigung oder Zerstörung von Sinneszellen des Innenohrs im Zusammenhang stehen.

Die Regeneration der Sinneszellen des Innenohrs erfolgt vorzugsweise durch eine Stimulation der Proliferation von Stützzellen des Innenohrs, das heißt der ebenfalls im Sinnesepithel, meist zwischen und unterhalb der Sinneszellen angeordneten Stützzellen. Da einer oder mehrere Zellzyklus-Inhibitoren in den Stützzellen des Innenohrs zu finden sind, kann durch die Hemmung oder Ausschaltung deren inhibierender Wirkung durch einen geeigneten Wirkstoff die Zellteilung der Stützzellen initiiert und damit eine Grundvoraussetzung für die Schaffung von Ersatzzellen für abgestorbene Sinneszellen geschaffen werden. Die aus der Teilung der Stützzellen hervorgehenden Zellen können dann mindestens teilweise zu funktionsfähigen Sinneszellen ausdifferenzieren.

Bei den bisher genannten Sinneszellen des Innenohrs handelt es sich vorzugsweise um die sogenannten Haarsinneszellen oder kurz Haarzellen, die an ihrem oberen Ende haarähnliche Fortsätze, die sogenannten Stereozilien oder Sinneshärchen aufweisen. Diese Haarzellen befinden sich auf der Basilarmembran im sogenannten Corti-Organ und bilden in Form sogenannter äußerer Haarzellen und sogenannter innerer Haarzellen die eigentlichen Rezeptorzellen für die Schalltransduktion im Innenohr. Interessant für eine Regeneration sind sowohl die inneren als auch die äußeren Haarzellen, wobei eine Regeneration der äußeren Haarzellen aufgrund deren größerer Empfindlichkeit ein besonderes Anwendungsgebiet der Erfindung darstellt. Diejenigen Stützzellen, die den inneren oder äußeren Haarsinneszellen anatomisch besonders gut zugeordnet sind, kommen für einen Einsatz des erfindungsgemäß verwendeten Wirkstoffs in besonderer Weise in Frage. So befinden sich neben den äußeren Haarsinneszellen als Stützzellen die sogenannten Hensenzellen, und unterhalb der äußeren Haarsinneszellen die sogenannten Deiterszellen und die "äußeren" Pillarzellen. Diese Hensenzellen, Deiterszellen und äußeren Pillarzellen sind dementsprechend als Ersatzzellen für die äußeren Haarsinneszellen besonders geeignet. In entsprechender Weise befinden sich neben und unterhalb der inneren Haarsinneszellen die sogenannten inneren Sulcuszellen als Stützzellen und unterhalb der inneren Haarsinneszellen auch die inneren Pillarzellen, die dementsprechend beide als Ersatzzellen für die inneren Haarsinneszellen in Betracht kommen. Dementsprechend kann ggf. eine Regeneration von inneren oder äußeren Haarzellen selektiv initiiert und beeinflußt werden. Hier kann auch auf die einschlägigen Fachbücher und Fachartikel über den Hörvorgang bei Säugern, insbesondere beim Menschen verwiesen werden. Die Regeneration der an der Schalltransduktion im Innenohr beteiligten Haarsinneszellen zur Behandlung der Innenohrschwerhörigkeit bei einer vorliegenden Schädigung dieser Sinneszellen wird durch die vorliegende Erfindung ermöglicht.

Bei den Zellzyklus-Inhibitoren, die erfindungsgemäß in ihrer inhibierenden Wirkung gehemmt oder ausgeschaltet werden, kann es sich grundsätzlich um verschiedene physiologische Stoffe, insbesondere Proteine handeln, die verhindern, daß die Zelle den normalen Zellzyklus einschließlich Zellteilung durchlaufen kann. Vorzugsweise handelt es sich um die sogenannten cyclin-dependent kinase inhibitors (CDKIs), also um Zyklinkinase-Inhibitoren. Von diesen ist bekannt, daß sie während der Entwicklung eines Organismus bei der Entstehung von terminal differenzierten Zellen verstärkt exprimiert werden und auf diese Weise einen Wiedereintritt der Zelle in den Zellzyklus verhindern. Dies würde auch den Verlust der Teilungsfähigkeit solcher Zellen mit verstärkter Exprimierung von Zyklinkinase-Inhibitoren erklären. Zellzyklus-Inhibitoren und dabei insbesondere Zyklinkinase-Inhibitoren der sogenannten CIP/KIP-Familie können selektiv in bestimmten Zelltypen exprimiert werden. Als bevorzugte Zyklinkinase-Inhibitoren sind hier insbesondere die als p21^{Cip1}, p27^{Kip1} und p57^{Kip2} bezeichneten Proteine zu nennen, wobei es sich erfindungsgemäß vorzugsweise um den Zyklinkinase-Inhibitor p27^{Kip1} handelt. Aufgrund der selektiven Exprimierung solcher Inhibitoren und der daraus resultierenden unterschiedlichen Expressionsmuster kann die Erfindung zur selektiven Beeinflussung des Zellzyklus in einem bestimmten Zelltyp eingesetzt werden. Wird beispielsweise in einem bestimmten Zelltyp, wie beispielsweise den Stützzellen im Sinnesepithel des Innenohrs, selektiv oder doch zumindest mit einem beträchtlichen Anteil p27^{Kip1} exprimiert, kann durch einen speziell auf diesen Inhibitor abgestellten Wirkstoff, dessen inhibierende Wirkung ausgeschaltet und damit die Proliferation der Stützzellen initiiert bzw. stimuliert werden. Über eine Ausdifferenzierung mindestens eines Teils der aus der Teilung der Stützzellen hervorgegangenen Zellen wird dann die Regeneration der Sinneszellen bewirkt.

Wie aus den bisherigen Ausführungen bereits hervorgeht, handelt es sich bei der Erkrankung oder Störung des Innenohrs erfindungsgemäß insbesondere um eine sogenannten Innenohrschwerhörigkeit. Diese steht mit der bereits erläuterten Schädigung oder Zerstörung der Haarsinneszellen im Innenohr im Zusammenhang.

Bei dem erfindungsgemäß einsetzbaren Wirkstoff, der den Zellzyklus-Inhibitor wiederum in seiner inhibierenden Wirkung hemmt oder ausschaltet, handelt es sich vorzugsweise um eine Substanz, die, üblicherweise intrazellulär, direkt oder indirekt auf den Inhibitor, das heißt üblicherweise ein Peptid oder Protein einwirkt. Dabei kann der Wirkstoff vorzugsweise als Peptid oder Protein vorliegen, das mit dem Inhibitor eine Peptid-Peptid- oder Protein-Protein-Wechselwirkung eingeht. Das wäre dann der Fall einer "direkten" Beeinflussung der Funktion des Inhibitors. Wenn als Wirkstoff ein Nukleinsäuremolekül verwendet wird, das für die Aminosäuresequenz eines oben genannten Peptids/Proteins kodiert, kann man von einer "indirekten" Beeinflussung sprechen, da zunächst das kodierende Nukleinsäuremolekül in die entsprechende Zelle eingeschleust und anschließend das (direkt als Wirkstoff dienende) Peptid-/Proteinmolekül exprimiert wird. Bei dem genannten Nukleinsäuremolekül kann es sich insbesondere um ein rekombiniertes Nukleinsäuremolekül handeln. Das Nukleinsäuremolekül kann grundsätzlich ein DNA-Molekül, ein cDNA-Molekül oder ein RNA-Molekül sein.

Ein weiterer Wirkstoff, der nach der Erfindung bevorzugt einsetzbar ist, ist ein Nukleinsäuremolekül, bei dem man sich die sogenannte Antisense-Technik zunutze macht. Bei dieser dem Fachmann grundsätzlich bekannten Technik wird üblicherweise eine RNA verwendet, die zur RNA des normalen (physiologischen) Gens komplementär ist. Diese komplementäre RNA nennt man Antisense-RNA. Die Antisense-RNA kann die Synthese des zu dem Gen gehörenden Proteinprodukts verhindern. Dies bedeutet im Fall der Erfindung, daß ein Nukleinsäuremolekül, beispielsweise die Antisense-RNA selbst oder die DNA, bei deren Transkription die Antisense-RNA entsteht, zur Hemmung oder Ausschaltung der inhibierenden Wirkung des Zellzyklus-Inhibitors in den Organismus bzw. die Zelle eingebracht wird. Dieses Einbringen kann vorzugsweise mit Hilfe von Lipidverbindungen geschehen, die zusätzlich virale Komponenten zum besseren Andocken und Eindringen des Nukleinsäuremoleküls in die Zelle tragen.

Wie geschildert kann der Wirkstoff bei der Erfindung eine unmittelbare Wechselwirkung, vorzugsweise eine Peptid-Peptid- oder Protein-Protein-Wechselwirkung mit dem Zelizyklus-Inhibitor eingehen. Der Wirkstoff kann jedoch auch mittelbar die inhibierende Wirkung des Zellzyklus-Inhibitors hemmen oder ausschalten, indem er mit einem physiologischen Wechselwirkungspartner des Zellzyklus-Inhibitors mindestens genauso gut oder vorzugsweise besser wechselwirkt als der Zellzyklus-Inhibitor selbst. Auf diese Weise wird dann verhindert, daß der Zellzyklus-Inhibitor seine physiologische (inhibierende) Wirkung entfalten kann.

So ist es beispielsweise im Falle des Zyklinkinase-Inhibitors p27^{Kip1} bekannt, daß dieser zusammen mit der zyklinabhängigen Kinase CDK2 und dem Zyklin A einen Proteinkomplex ausbildet. Dabei existieren bestimmte Stellen, an denen zwischen dem p27^{Kip1} und CDK2 bzw. Zyklin A Peptid-Peptid-Wechselwirkungen stattfinden. So wurde beispielsweise eine Bindungsstelle sehr hoher Affinität zwischen p27^{Kip1} und Zyklin A identifiziert und mehrere weniger starke Bindungsstellen zwischen p27^{Kip1} und Zyklin A bzw. p27^{Kip1} und CDK2. Greift man nun eine der Bindungsstellen heraus, an denen keine Bindung/Interaktion mit hoher oder sehr hoher Affinität vorliegt, so kann ein Wirkstoff, vorzugsweise in Form eines weiteren Peptids/Proteins ausgewählt oder entwickelt werden, der mit einem der beiden Wechselwirkungspartner an der betreffenden Bindungsstelle eine Bindung/Interaktion mindestens gleich hoher oder vorzugsweise höherer Affinität eingeht. Dadurch wird dann die übliche physiologische Wechselwirkung gehemmt oder verhindert, da die entsprechende Bindungsstelle für den physiologischen Wechselwirkungspartner blockiert ist.

So kann beispielsweise für eine Bindungsstelle zwischen p27^{Kip1} und Zyklin A, aber auch CDK2, eine optimierte Peptidstruktur oder optimierte Aminosäuresequenz für die Aminosäuresequenz des p27^{Kip1} an dieser Stelle entwickelt werden, die dann an die entsprechende Sequenz des Zyklin A bzw. CDK2 an dieser Stelle mit besserer, das heißt höherer Affinität bindet. Eine derartige optimierte Peptidstruktur umfaßt beispiels- und vorzugsweise bis zu 15 Aminosäuren und kann dann direkt in die Zelle eingebracht oder vorzugsweise durch ein künstlich eingebrachtes Gen intrazellulär exprimiert werden. Durch die hohe Affinität eines solchen Peptids wird dann die Wechselwirkung der physiologischen Peptidpartner gestört und die Ausbildung des Peptidkomplexes, auf der die inhibierende Wirkung des Zellzyklus-Inhibitors beruht, verhindert. Auf diese Weise sorgt dann der Wirkstoff für eine mindestens teilweise Hemmung oder eine vollständige Ausschaltung der inhibierenden Wirkung des Zellzyklus-Inhibitors. Durch diesen Verfahrensansatz der Erfindung muß die Konzentration des Wirkstoffs, insbesondere des Peptids/Proteins mit der entsprechenden Aminosäuresequenz in der Zelle nur etwa gleich hoch sein wie die entsprechende Konzentration des Zellzyklus-Inhibitors, der in seiner Wirkung gehemmt oder ausgeschaltet werden soll. Da derartige Konzentrationen, beispielsweise von p27^{Kip1} in der Größenordnung von ca. 10 nM/l liegen und etwa 1000 bis 10000 Molekülen pro Zelle entsprechen, können bereits sehr niedrige Konzentrationen für die Durchführung der Erfindung ausreichen. Wichtig ist ebenfalls, daß zum Erreichen einer solchen Konzentration mit Hilfe gentherapeutischer Methoden bereits das Einschleusen einer einzigen Kopie einer DNA, die für die entsprechende Aminosäuresequenz kodiert, pro Zelle ausreicht. Dies stellt gegenüber anderen Methoden, die mit viel höheren Konzentrationen oder einer größeren Vielzahl von DNA-Kopien arbeiten müssen, einen entscheidenden Vorteil dar.

In Weiterbildung kann die erfindungsgemäße Verwendung so durchgeführt werden, daß der Wirkstoff in Form eines sogenannten Vektors oder Vehikels vorliegt, wobei dieser Vektor oder dieses Vehikel mindestens eines der bereits beschriebenen Nukleinsäuremoleküle trägt. Vorzugsweise handelt es sich dabei um ein Nukleinsäuremolekül, das für die Aminosäuresequenz eines als Wirkstoff dienenden Peptids oder Proteins kodiert. Bei den genannten Vektoren kann es sich um die üblichen viralen und nicht-viralen Vektoren handeln, wie sie dem Fachmann bekannt sind. So können in üblicherweise Retroviren, Adenoviren oder adeno-assoziierte Viren als virale Vektoren eingesetzt werden. Bei nicht-viralen Vektoren ist bekanntermaßen keine virale DNA beteiligt, so daß hier grundsätzlich eine "nackte" DNA in eine Zelle eingebracht werden kann. Vorzugsweise werden derartige Nukleinsäuremoleküle jedoch üblicherweise in sogenannte Liposomen oder Lipoplexe verpackt sein und in dieser Form in den Organismus und die Zelle eingebracht werden. Der Einsatz von nicht-viralen Vektoren bzw. Lipoplexen ist grundsätzlich bevorzugt, da virale Vektoren einige, dem Fachmann bekannte Nachteile aufweisen. Aufgrund der soeben geschilderten Einsatzmöglichkeiten der Erfindung kann hier häufig ohne Verwendung viraler Vektoren gearbeitet werden, da die Effektivität der verwendeten Wirkstoffe selbst sehr hoch ist und dementsprechend mit niedrigen Konzentrationen gearbeitet werden kann.

Bei der Erfindung wird der verwendete Wirkstoff vorzugsweise in einer therapeutisch wirksamen Menge eingesetzt. Dieser kann in üblicher Weise auf das zu behandelnde Subjekt abgestellt sein, wobei u.a. bekannte pharmazeutische Zusatzstoffe zum Einsatz kommen können. In Weiterbildung kann der verwendete Wirkstoff zur lokalen Applikation vorgesehen sein. Damit können mögliche Nachteile einer systemischen Applikation vermieden werden. Der Zielort des erfindungsgemäßen Verfahrens, nämlich das Innenohr, ist für eine lokale Applikation in besonderem Maße geeignet. So kann der Wirkstoff im vorliegenden Fall in den sogenannten Perilymphraum des Innenohrs des Säugers, insbesondere des Menschen eingebracht werden. Hier handelt es sich um einen kleinen Flüssigkeitsraum mit sehr langsamer Austauschgeschwindigkeit, der einer therapeutischen Intervention vom Mittelohr her, beispielsweise über die Membran des runden Fensters, zugänglich ist. Dieser Perilymphraum hat ein Volumen von nur etwa 20 Mikrolitern und steht zudem in direktem Kontakt mit den Zellen des Corti-Organs. Damit ist ein direktes Einwirken des Wirkstoffes auf das Sinnesepithel mit seinen Haarzellen und Stützzellen gewährleistet.

Der Wirkstoff ist zur Regeneration der Sirineszellen des Innenohrs, insbesondere der Haarsinneszellen des Innenohrs ausgebildet und in der Lage, einen im Innenohr vorhandenen sogenannten Zellzyklus-Inhibitor in seiner (inhibierenden) Wirkung mindestens teilweise zu hemmen oder auszuschalten. Bei dem Zellzyklus-Inhibitor handelt es sich vorzugsweise um einen Zyklinkinase-Inhibitor, insbesondere den Zyklinkinase-Inhibitor p27^{Kip1}. Bezüglich der genauen und bevorzugten Eigenschaften des Wirkstoffs wird auf die bisherigen Ausführungen Bezug genommen und verwiesen. Wie geschildert kann es sich hierbei vorzugsweise um mindestens ein Peptid/Protein oder um mindestens ein Nukleinsäuremolekül handeln, wobei letzteres vorzugsweise eine Antisense-DNA oder Antisense-RNA ist oder vorzugsweise für ein entsprechendes als Wirkstoff einsetzbares Peptid/Protein kodiert. Bei dem Nukleinsäuremolelkül kann es sich um ein DNA-Molekül, ein cDNA-Molekül oder ein RNA-Molekül handeln. Insbesondere das Nukleinsäuremolekül wird mit Hilfe eines geeigneten Vektors oder Vehikels in den Organismus bzw. die Zelle eingebracht, wobei es sich um die beschriebenen viralen oder nicht-viralen Vektoren bzw. um in Liposomen/Lipoplexen verpackte Nukleinsäuremoleküle handeln kann.

Die beschriebenen Merkmale und weitere Merkmale der Erfindung ergeben sich aus der nachfolgenden Beschreibung einer bevorzugten Ausführungsform in Verbindung mit den Unteransprüchen, dem Beispiel und der Zeichnung. Hierbei können die einzelnen Merkmale jeweils einzeln für sich oder zu mehreren in Kombination miteinander verwirklicht sein.

In der Zeichnung zeigt:

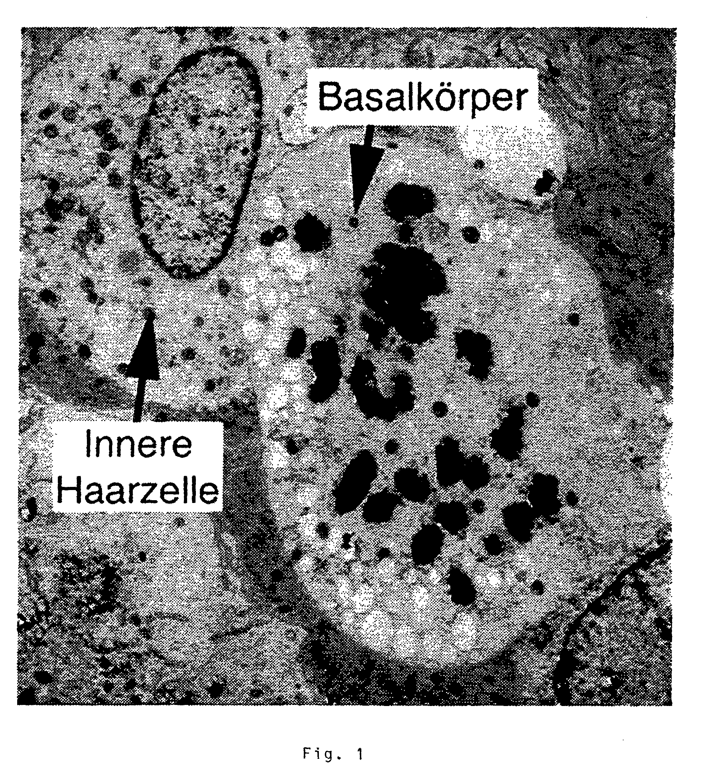

- Figur 1: die elektronenmikroskopische Aufnahme einer in Kernteilung befindlichen Zelle im sensorischen Epithel des Corti-Organs einer sogenannten p27^{Kip}-Knockout-Maus.

### Beispiel

Für den Versuch wird eine sogenannte p27^{Kip1}-Knockout-Maus (p27⁻/₋) verwendet, das heißt eine Maus, der das Gen zur Expression des Proteins p27^{Kip1} fehlt. Dementsprechend kann in einer solchen Maus p27^{Kip1} seine Zellzyklus-inhibierende Wirkung per se nicht entfalten.

Von einer solchen p27^{Kip1}-Knockout-Maus wird am siebten Tag nach der Geburt (postnataler Tag 7) das Cortische Organ entnommen und in üblicher Weise für eine elektronenmikroskopische Untersuchung derart präpariert, daß das sensorische Epithel des Cortischen Organs zu sehen ist.

Das Ergebnis der elektronenmikroskopischen Untersuchung ist in Figur 1 dargestellt. In dieser elektronenmikroskopischen Aufnahme ist zu erkennen, daß zwischen zwei links oben bzw. rechts unten befindlichen inneren Haarzellen, von denen die schwarz umrandeten Zellkerne links oben (vollständig) und rechts unten (teilweise) zu erkennen sind, eine in Kernteilung (Mitose) befindliche Zelle, das heißt eine mitotische Zelle angeordnet ist. Die Mitose ist eindeutig erkennbar am kondensierten Chromatin, der aufgelösten Kernmembran und dem Basalkörperchen. Die links oben angeordnete innere Haarzelle und das Basalkörperchen sind in der Figur zur besseren Erkennbarkeit mit englichsprachiger Beschriftung bezeichnet.

Damit ergibt sich aus Figur 1 eindeutig, daß das Fehlen des Zellzyklus-Inhibitors p27^{Kip1} dazu führt, daß im Sinnesepithel des Cortischen Organs der Maus eine Zellteilung von dort angeordneten Stützzellen möglich ist. Hierbei ist noch zu erwähnen, daß es sich bei der in Figur 1 dargestellten Zellteilung nicht um eine Einzelerscheinung innerhalb des sensorischen Epithels des Corti-Organs handelt, sondern daß sehr viele dort befindliche Zellen eine Zellteilung und damit den Zellzyklus durchlaufen. Die in Figur 1 dargestellte Erscheinung läßt den Schluß zu, daß nicht nur eine Zellteilung, sondern nach einer Zellteilung, die den entscheidenden Schritt im Haarzellregenerationsprozess darstellt, im weiteren auch eine Differenzierung zu Haarsinneszellen und schließlich eine funktionelle Erholung der Hörfunktion des Sinnesorgans eintritt. Damit ist eine Regeneration der Sinneszellen möglich. Für die Zulässigkeit dieses Schlusses spricht auch, daß bei der Knockout-Maus nicht nur eine Mitose stattfindet, sondern daß derartige Knockout-Mäuse auch mehr Haarzellen besitzen als normale Mäuse, bei denen das Protein p27^{Kip1} exprimiert wird. Dementsprechend resultiert die Mitose der Stützzellen auch in ausdifferenzierten Sinneszellen. Die Richtigkeit dieses Schlusses wird durch die folgenden Ergebnisse bestätigt. Bei heterozygoten Knockout-Mäusen wurde die Regeneration der Haarzellen dadurch nachgewiesen, daß in der zweiten Lebenswoche der Tiere, wenn diese die Hörfunktion entwickeln, die Haarzellen durch systemische Verabreichung von Amikacin zerstört wurden. Nach weiteren zwei Wochen ohne jede Injektion wurden die Tiere getötet und ihre Cochlea untersucht. Dabei zeigten sich neu gebildete Haarzellen in der Cochlea, die durch einen, beispielsweise mit dem Amikacin verabreichten, Proliferationsmarker (Bromdesoxyuridin (BrdU)) markiert sind.

Als Konsequenz daraus läßt sich nicht nur bei Knockout-Mäusen, denen das Gen für p27^{Kip1} von vornherein fehlt, sondern auch durch eine Hemmung oder Ausschaltung des im normalen Organismus exprimierten p27^{Kip1}, beispielsweise mit Hilfe eines mit dem p27^{Kip1} oder einem seiner physiologischen Partner wechselwirkenden Peptids, mit Hilfe der für dieses Peptid kodierenden Nukleinsäuresequenz oder mit Hilfe einer Antisense-DNA/Antisense-RNA eine Regeneration der Sinneszellen erreichen. Dies kann auch durch eine nur teilweise Ausschaltung der Funktion von p27^{Kip1} geschehen, da bei den heterozygoten Mäusen ein Gen-Dosis abhängiger Effekt beobachtet wird.

## Patentansprüche

1. Verwendung eines Wirkstoffs, der in der Lage ist, mindestens einen im Innenohr vorhandenen Zyklinkinase-Inhibitor in seiner inhibierenden Wirkung mindestens teilweise zu hemmen oder auszuschalten, zur Herstellung einer pharmazeutischen Zusammensetzung oder eines Medikaments zur Behandlung von Erkrankungen oder Störungen des Innenohrs, die mit einer Schädigung oder Zerstörung von Sinneszellen des Innenohrs im Zusammenhang stehen, durch Regeneration von Sinneszellen im Innenohr.

2. Verwendung nach Anspruch 1, **dadurch gekennzeichnet, dass** es sich bei dem Zyklinkinase-Inhibitor um den Zyklinkinase-Inhibitor p27^{Kip1} handelt.

3. Verwendung nach Anspruch 1 oder Anspruch 2, **dadurch gekennzeichnet, dass** es sich bei dem Wirkstoff um mindestens ein Nukleinsäuremolekül, insbesondere rekombiniertes Nukleinsäuremolekül, handelt.

4. Verwendung nach Anspruch 3, **dadurch gekennzeichnet, dass** das Nukleinsäuremolekül ein RNA-Molekül ist.

5. Verwendung nach Anspruch 3 oder 4, **dadurch gekennzeichnet, dass** das Nukleinsäuremolekül eine Antisense-Sequenz ist.

## Claims

1. A use of an active agent capable of at least partially inhibiting or eliminating at least one cyclin kinase inhibitor present in the inner ear in its inhibitive action, for the preparation of a pharmaceutical composition or a medicament for the treatment of diseases or disorders of the inner ear related to a damage or destruction of sensory cells of the inner ear by regeneration of sensory cells in the inner ear.

2. The use according to claim 1, **characterized in that** the cyclin kinase inhibitor is the cyclin kinase p27^{Kip1}.

3. The use according to claim 1 or claim 2, **characterized in that** the active agent is at least one nucleic acid molecule, in particular a recombinant nucleic acid molecule.

4. The use according to claim 3, **characterized in that** the nucleic acid molecule is an RNA molecule.

5. The use according to claim 3 or 4, **characterized in that** the nucleic acid molecule is an antisense sequence.

## Revendications

1. Utilisation d'un agent actif capable d'au moins partiellement inhibiter ou eliminer l'action inhibitive d'au moins un inhibiteur de kinase de la cycline présent dans l'oreille interne, pour la préparation d'une composition pharmaceutique ou d'un médicament pour le traitement de maladies ou désordres de l'oreille interne en rapport avec une lésion ou destruction de cellules sensorielles de l'oreille interne par régénération de cellules sensorielles dans l'oreille interne.

2. Utilisation selon la revendication 1, caracterérisée en ce que l'inhibiteur de kinase de la cycline est l'inhibiteur de kinase de la cycline p27^{Kip1}.

3. Utilisation selon la revendication 1 ou la revendication 2, **caractérisée en ce que** l'agent actif est au moins une molécule d'acide nucléique, notamment molécule d'acide nucléique recombinée.

4. Utilisation selon la revendication 3, **caractérisée en ce que** la molécule d'acide nucléique est une molécule d'ARN.

5. Utilisation selon la revendication 3 ou 4, **caractérisée en ce que** la molécule d'acide nucléique est une séquence antisens.
